# EUROPÄISCHE PATENTSCHRIFT

(11) **EP 0 595 892 B1**
(45) Veröffentlichungstag und Bekanntmachung des Hinweises auf die Patenterteilung: **20.12.1995**
(21) Anmeldenummer: 92915550.5
(22) Anmeldetag: 21.07.1992
(51) Int. Cl.: A61B 17/04

(54) **CHIRURGISCHES NÄHINSTRUMENT**
SURGICAL STITCHING INSTRUMENT
INSTRUMENT DE SUTURE CHIRURGICAL

(30) Priorität: 23.07.1991 DE 4124381; 23.07.1991 DE 4124383; 30.11.1991 DE 4139628
(43) Veröffentlichungstag der Anmeldung: 11.05.1994
(73) Patentinhaber: Forschungszentrum Karlsruhe GmbH, 76133 Karlsruhe (DE)
(72) Erfinder: MELZER, Andreas, D-6200 Wiesbaden (DE); BUESS, Gerhard, D-7400 Tübingen (DE); TRAPP, Rainer, D-7523 Graben-Neudorf (DE); BRHEL, Klaus-Peter, D-7522 Philippsburg (DE)
(74) Vertreter: Gottlob, Peter, Dipl. Ing.
(86) Internationale Anmeldenummer: DE9200589
(87) Internationale Veröffentlichungsnummer: WO9301750

(56) Entgegenhaltungen:
- EP-A- 0 174 843
- EP-A- 0 315 371
- DE-A- 2 447 719
- DE-C- 4 124 383
- US-A- 2 880 728
- US-A- 3 692 224
- US-A- 4 236 470
- US-A- 4 836 205
- US-A- 4 935 027

## Beschreibung

Die Erfindung betrifft ein chirurgisches Nähinstrument, mit dem eine Naht an einer Operationsstelle endoskopisch gelegt werden kann.

Sinn und Zweck derartiger Instrumente ist es, die für den Eingriff am zu operierenden Körper erforderliche Verletzung auf ein notwendiges Minimum zu beschränken und weiter den Chirurgen in seiner operativen Tätigkeit zu entlasten. Diese Technik wird unter dem Begriff minimal invasive Chirurgie (MIC) gefaßt.

Aus der EP 0 174 843 A2 ist eine Näh- und Heftmaschine bekannt, die über ein Instrumentenkanal in den Körper eines Patienten einführbar und von außen bedienbar ist. Der Nähkopf der Maschine ist zur Seite hin geöffnet. Dort endet ein Saugrohr, über das Gewebe angesaugt werden kann, so daß eine Gewebefalte zustande kommt. Eine Nadel mit eingefädeltem Nähfaden, bewegt sich dann parallel zur Maschinenachse und durchsticht das Gewebe. Der mitgeführte Faden kann über eine Fangvorrichtung schleifenbildend gehalten werden. Eine Knotung erfolgt gesondert.

Die US 4,935,027 offenbart ein chirurgisches Instrument als auch ein Verfahren, mit dem von entfernter Position oder endoskopisch genäht werden kann. Die Zangen des Instruments sind hohl und bilden in geschlossenem Zustand eine Öse, durch die ein Nähfaden geschoben oder transportiert werden kann. Die Zangenenden sind derart gestaltet, daß sie trotz Höhlung zu nähende Substanzen durchstechen. Nach jedem Nähschritt oder dem Nähvorgang überhaupt erfolgt die Knotenanlegung z.B. von außen und wird dann zum Knoten geschoben. Auf jeden Fall sind zusätzliche Instrumente dazu notwendig.

Die US 4,836,205 beschreibt ein Greif- und Nähinstrument, das besonders für die arthroskopische Kniechirurgie konzipiert wurde. Zu diesem Zweck ist am distalen Ende des Instruments ein Greifvorrichtung, um zerissenes Gewebe halten zu können. Die Greifvorrichtung besteht aus einem beweglichen und einem feststehenden, geschwungenen Maulteil, das in zusammengeklapptem Zustand einen axialen Freiraum aufweist, durch den eine chirurgische Nähnadel zum Durchdringen des festgehaltenen Gewebes vorgeschoben und zurückgezogen werden kann. Das Maulteil und die Nadel werden von einer Greifzange aus über einen Rohrantrieb betätigt.

Eine handbedientes, chirurgisches Nähinstrument wird in der US 4,236,470 beschrieben. Das Instrument ist pinzettenartig aufgebaut und weist an den beiden freien Enden der Schenkel je eine Halteeinrichtung auf. Eine chirurgische Nadel, deren beide Enden als Spitzen ausgebildet sind, und die ein Nadelöhr in der Mitte des Teils zwischen den Spitzen aufweist, kann von einer Halteeinrichtung in die andere umgesetzt werden. Die Halteeinrichtungen werden zu diesem Zweck verriegelt oder gelöst. Der Mechanismus hierzu ist den Schenkeln des Nähinstruments untergebracht und wird von Hand betätigt.

Die Akzeptanz eines solchen Nähinstruments durch den Chirurgen wird u.a. wesentlich durch den Grad der raschen Gewöhnung an dieses und durch die möglichst leichte, konfliktfreie Bedienbarkeit bestimmt.

Der Erfindung liegt die Aufgabe zugrunde, ein durch einen Instrumentenkanal einführbares und handhabbares Nähinstrument bereitzustellen, mit dem chirurgische Nähte an Gewebe oder Gefäßen und Knotungen weitestgehend ohne Zusatzinstrumente durchführbar sind. Diese Aufgabe wird durch die im Anspruch 1 gekennzeichneten Merkmale gelöst.

Hierzu weist das Nähinstrument an seinem fernen Ende einen Maulbereich auf der aus einem feststehenden und einem beweglichen Maulteil besteht, das durch diesen konstruktiven Aufbau bei einer nicht eingespannten Nähnadel als Greifinstrument gebraucht werden kann.

An den freien Enden der beiden Maulteile sind Klemmvorrichtungen eingebaut, in die die Nähnadel form- und definiert kraftschlüssig eingespannt bzw. eingeklemmt werden kann. Sie kann von einer Haltevorrichtung in die andere ohne ein weiteres Halteinstrument umgesetzt werden. Sie kann aber auch gleichzeitig in beide Haltevorrichtungen eingespannt werden.

Die Bewegung des beweglichen Maulteils erfolgt durch den Chirurgen einhändig am beweglichen Griffteil der Griffzange und damit auch das Herausziehen oder Eindrücken der Nadel aus oder in die Klemmvorrichtung desselben.

Hingegen wird das Einklemmen oder Freigeben der Nähnadel in oder von der Klemmvorrichtung pneumatisch über einen Pedalschalter durch den Fuß des Chirurgen bewirkt. Hierzu ist eine Pneumatikzuleitung an den Pedalschalter angeschlossen von dem aus eine Pneumatikleitung an den Anschlußstutzen eines Miniaturpneumatik-Zylinders an der Griffzange führt, der je nach Druckzustand in ihm über eine Hebelumlenkeinrichtung und einem Antriebsrohr durch den Instrumentenkanal die eine Hälfte form- und definiert kraftschlüssig anpreßt oder davon entfernt.

Der Stichvorgang durch das zu nähende Gewebe oder Gefäßteil und der Nähnadelumsetzvorgang sind dadurch klar voneinander getrennt. Der Chirurg hat somit die andere Hand ständig frei für eine eventuell notwendige Nachführung des Nähinstrumentes am Operationsort.

Die Unteransprüche geben eine vorteilhafte Ausgestaltung des Nähinstrumentes und der Griffzange wieder.

Beide Klemmvorrichtungen an den Maulteilen, weisen so vorteilhaft die Negativform der Nähnadelenden auf, die aus Spitze, Wulst und Taille bestehen. Der Übergang vom Wulst auf die Taille erfolgt kegelstumpfförmig oder pyramidenstumpfförmig entsprechend der Nadelspitzenform. Das Herausziehen und Eindrücken der Nadelspitze aus und in die Klemmvorrichtung am beweglichen Maulteil erfolgt dadurch beschädigungslos und kraftbestimmt bzw. kraftbeschränkt. Dasselbe ist für das Einspannen der Nadelspitze an der Klemmvorrichtung des feststehenden Maulteils gegeben, da der Druck im Miniaturpneumatikzylinder beschränkt gehalten werden kann und durch die Auslegung der Hebelumlenkeinrichtung die Anpreßkraft der beweglichen Hälfte an die feststehende Hälfte der Klemmvorrichtung einstellbar ist.

Für das Knotenlegen am Nähort ist es weiter vorteilhaft, wenn das bewegliche Griffzangenteil der Griffzange durch eine freigebbare Einrastvorrichtung festgestellt und durch Überziehen über einen Druckpunkt wieder freigegeben werden kann.

Je nach durchzuführender Operation und dabei durchzuführendem Nähvorgang ist es ebenfalls vorteilhaft, zwei konstruktiv verschiedene Ausführungsformen des Maulbereichs zur Verfügung zu haben. Die eine zeichnet sich dadurch aus, daß der Maulbereich des Nähinstrumentes aus zwei gleichartig senkrecht zur Nähinstrumentenachse stehenden Maulteilen besteht, wobei das bewegliche Maulteil axial über die Griffzange zum feststehenden Maulteil geschoben und davon wegbewegt werden kann, so daß eine einseitig oder beidseitig eingespannte Nähnadel parallel zur Instrumentenachse liegt. Zweckmäßigerweise ist die mit dieser Form des Nähinstrumentes verwendete Nadel gerade, damit Gewebs- oder Gefäßteile durch die Nadel nur gestochen und dabei nicht angerissen werden kann.

Die Klemmvorrichtung am feststehenden Maulteil besteht aus der einen feststehenden Hälfte und der anderen klappbaren Hälfte.

Diese klappbare Hälfte ist zu diesem Zweck in einem dort um einen vorgegebenen Winkel drehbar gelagerten Hebel eingebaut, der über den Pedalschalter betätigt wird.

Die andere konstruktive Ausführungsform des Nähinstruments besteht aus dem in Richtung Instrumentenachse ausgerichteten Maulbereich. Das bewegliche Maulteil ist um eine Achse senkrecht zur Instrumentenachse drehbar gelagert und kann bis zu einem vorgegebenen Winkel aufgeklappt bzw. auf das feststehende Maulteil geklappt werden. Die Klemmvorrichtungen unterscheiden sich nicht zu denen von oben. Lediglich die bewegliche Hälfte am feststehenden Maulteil wird jetzt nicht auf die andere feststehende Hälfte geklappt, sondern auf sie zu- oder von ihr weggeschoben. Der Antrieb dazu geht wie oben von dem Pedalschalter aus.

Dieser Aufbau des Nähinstrumentes eignet sich besonders auch als Greifinstrumente. Es sind daher die freien Enden der Maulteile geeignet gerippt.

Um Gewebe- oder Gefäßeinrisse mit diesem Nähinstrument beim Nähvorgang zu vermeiden, ist die dazu verwendete Nähnadel so gekrümmt, daß ihre Achse mit der Bahn der Klemmvorrichtung am beweglichen Maulteil zusammenfällt.

Die beiden Formen, das heißt die beiden Hälften der jeweiligen Klemmvorrichtung sind austauschbar und können der Spritzenform der verwendeten Nadel entsprechend eingesetzt werden. Das hat den wirtschaftlichen Vorteil, daß ein Nähinstrument mit verschiedenen Nadelarten verwendet werden kann.

Dieser Vorteil des Aus- und Einbaus verschiedener Klemmvorrichtungen ist ausdehnbar in der Art, daß das Nähinstrument statt zum Nähen auch zum Klammern von Gefäß oder Gewebeteilen verwendet werden kann, sofern die Klemmvorrichtungen durch eine Klammerhaltevorrichtung und eine zugehörige Gesenkvorrichtung ausgetauscht wird.

Die Erfindung wird nachstehend anhand der in der Zeichnung dargestellten Ausführungsformen näher erläutert.

Es zeigt:
Figur 1 das Nähinstrument mit seitlich angebrachtem Maulbereich;
Figur 2 das Nähinstrument mit nach vorne gerichtetem Maulbereich;
Figur 3 die Griffzange zum Betätigen des Nähinstruments;
Figur 4 ein über Zahnkranz und Zahnstange angetriebenes Maulteil am Nähinstrument nach Figur 2;
Figur 5 eine Nähnadel für das Nähinstrument nach Figur 1;
Figur 6 einen Längsschnitt durch die Nähnadel;
Figur 7 die in eine Klemmvorrichtung eingeklemmte Nähnadel.

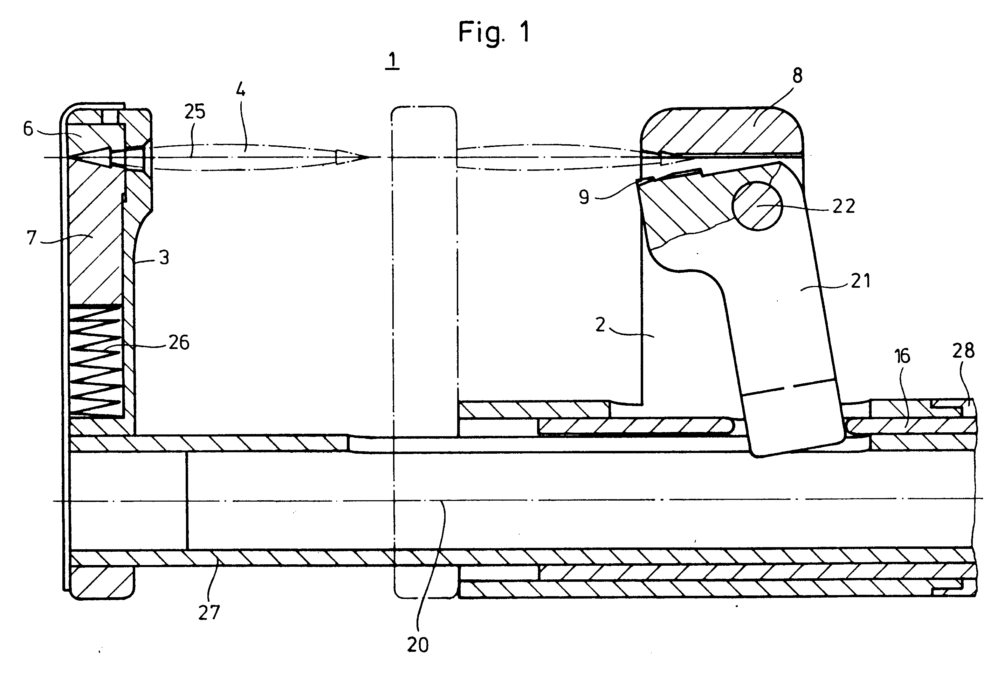

In Figur 1 ist der Nähkopf 1 des von Hand fernbedienten chirurgischen Nähinstruments in Seitenansicht dargestellt. Er besteht aus den zwei Maulteilen 2, 3 die gleichgerichtet senkrecht von der Achse 20 des Nähinstruments abstehen.

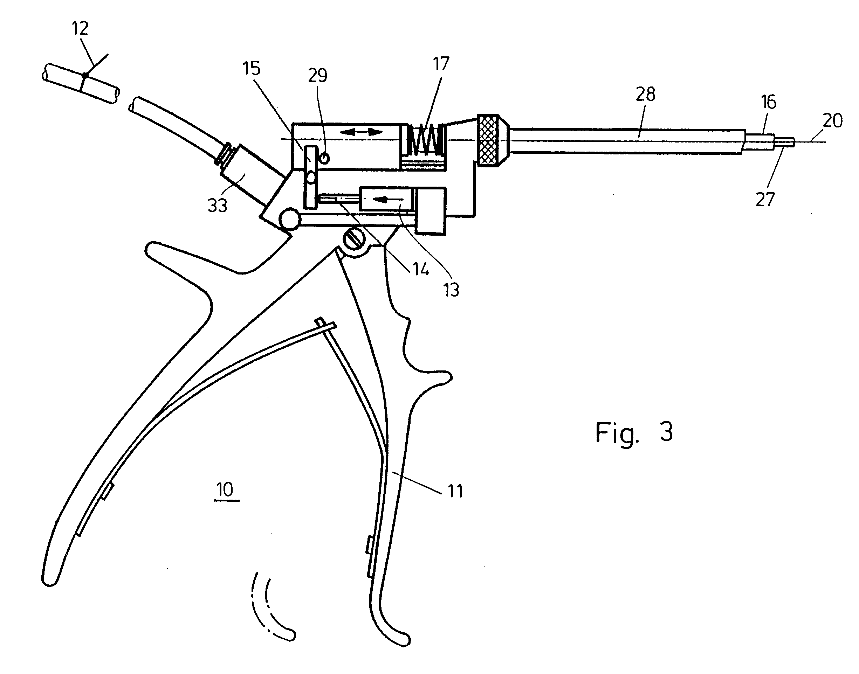

Am freien Ende des Maulteils 3 befindet sich der Einsatz der Klemmvorrichtung 6, 7, der pfeilspitzenförmig vertieft ist. Die Achse 25 dieser Klemmvorrichtung 6, 7 liegt parallel zur Nähinstrumentenachse 20. Die Klemmvorrichtung 6, 7 kann von der freien Kopfseite her am Maulteil 3 aus- und eingebaut werden. Sie besteht aus zwei Hälften 6, 7. Die eine Hälfte 6 ist feststehend. An sie drückt sich über eine Feder 7 mit vorbestimmter Federkonstanten die andere bewegliche Hälfte 7 und bildet aneinandergepreßt die Negativform der Nadelspitze 5. Dadurch ist die Nadel form- und kraftschlüssig eingespannt. Es wird so auf die eingeklemmte Nadelspitze eine axiale und radiale Kraftwirkung um den gesamten Umfang herum ausgeübt wird. Die Nadel läßt sich über einen gewissen axialen Kraftaufwand aus dieser Klemmvorrichtung herausziehen. Das Maulteil 3 ist fest mit dem koaxialen Rohr 27 verbunden und in axialer Richtung hin- und her beweglich. Bewirkt wird das durch Betätigen des beweglichen Teils der Griffzange (siehe Figur 3).

Gegenüber am feststehenden Maulteil 2, befindet sich die andere feststehende Klemmvorrichtung 8, 9 für die Nadel 4. Diese Klemmvorrichtung 8, 9 kann über den drehbar gelagerten Hebel 21 geschlossen oder geöffnet werden. Der Hebel 21 dreht dabei um die Drehachse 22, wodurch die bewegliche Hälfte 9 der Klemmvorrichtung 8, 9 an die feststehende Hälfte 8 angedrückt oder von ihr weggedreht wird. Betätigt wird der Hebel 21 über das Rohr 16, das wiederum von der Antriebseinrichtung 13, 14, 15 an der Griffzange 10 bewegt wird.

Dieser Aufbau ermöglicht dadurch ein müheloses Umsetzen der Nähnadel 4 von einer Klemmvorrichtung 6, 7 in die andere 8, 9 oder gar ein gleichzeitiges Einklemmen beider Nadelspitzen 5. Form- und definierter Kraftschluß in beiden Klemmvorrichtungen 6, 7; 8, 9 halten die Nadel in jedem Fall in der vorgesehenen Lage.

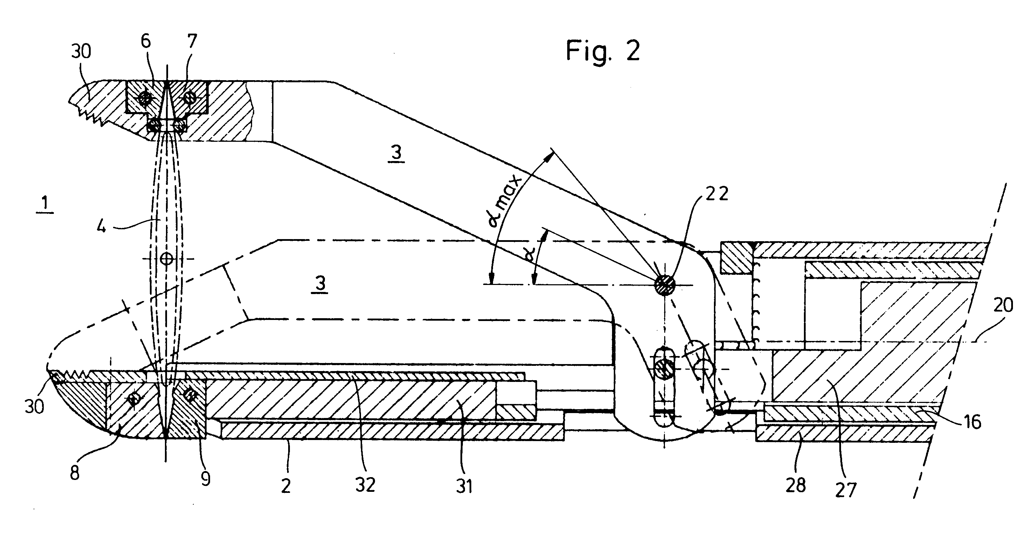

Konstruktiv anders ausgerichtet ist der Nähkopf 1 in Figur 2. Die Öffnung der Maulteile 2, 3 sind von der Instrumentenachse aus gesehen nach vorne gerichtet. Das starre Maulteil 2 liegt parallel, d.h. neben der Instrumentenachse 20. Das andere, bewegliche Maulteil 3 läßt sich auf- und zuklappen und ist an der Gelenkachse 22 gelagert. Es läßt sich um einen konstruktiv vorgegebenen Winkel α drehen. Hierzu muß ein koaxiales Rohr 16 im Instrumentenrohr 28 hin- und herbewegt werden, das am Ende über einen Bolzen 29, 30 mit dem Maulteil 3 gekoppelt ist. Ist in einer der Klemmvorrichtungen 6, 7; 8, 9 keine Nadel 4 eingespannt, so läßt sich das bewegliche Maulteil 3 so zuklappen, daß sich die beiden gerippten Flächen 30 schlüssig ineinanderlegen. Der Nähkopf 1 des Nähinstruments ist somit auch als Greifeinrichtung verwendbar. Der gestrichelte Umriß des Maulteils 3 ist in dieser Stellung gezeichnet.

Am fernen Ende der beiden Maulteile 2, 3, unmittelbar hinter den gerippten Flächen 30 ist je eine, jederzeit separat lösbare Klemmvorrichtung 6, 7; 8, 9 eingebaut. Beide Klemmvorrichtungen 6, 7; 8, 9 haben, entsprechend der Negativform der Nadelspitze 5, eine Vertiefung, die einen runden oder dreieckigen oder mehreckigen Querschnitt haben. Letztere sind insbesondere eine Sicherung gegen axiales Verdrehen der in der Klemmvorrichtung eingespannten Nadel.

Die Klemmvorrichtung 8, 9 in dem feststehenden Maulteil 2 besteht wie in Figur 1 aus den zwei Hälften 8, 9. Die eine Hälfte 8 ist fest mit dem Maulteil 2 verbunden. Die andere Hälfte 9 ist am Kopf des Rohres 16 über die Schiebevorrichtung 31 montiert und läßt sich mit diesem hin- und herschieben, so daß diese Klemmvorrichtung 8, 9 geöffnet und geschlossen werden kann. Die Nähnadel 4 kann so auch hier form- und kraftschlüssig eingespannt werden. Die Betreibung von der Griffzange 10 her erfolgt, wie schon zu Figur 1 erläutert.

Es hat sich ebenfalls als zweckmäßig herausgestellt, die Schiebevorrichtung 31 zwischen beweglicher Hälfte 9 und Ende des Antriebsrohres 27 gegen den Raum zwischen den beiden Maulteilen mit einem Blech 32 abzudecken.

Die Klemmvorrichtung 6, 7 am beweglichen Maulteil 3 ist identisch mit der am entsprechenden Maulteil in Figur 1 aufgebaut und daher in Funktion und Wirkung gleich.

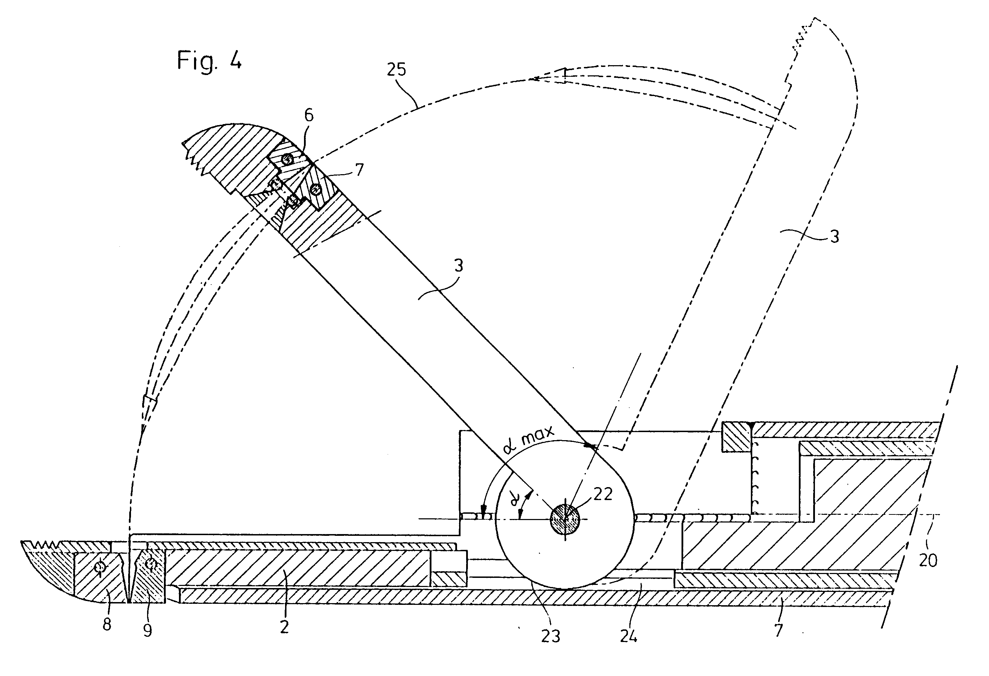

Die gestrichelt eingezeichnete Nadel 4 ist in dieser Bauweise gerade. Das ist für kleine maximale Öffnungswinkel α max unproblematisch vom chirurgischen Standpunkt. Für größere öffnungswinkel α max, die die beiden Maulteile miteinander bilden können, ist es, um Gewebe- oder Gefäßrisse zu vermeiden, vorteilhaft, wenn die Nähnadel 4 gekrümmt ist (siehe unten Beschreibungsteil zu Figur 4).

Die Nähköpfe aus den beschriebenen Figuren 1 und 2 können über eine Griffzange 10 betrieben werden. Hiervon werden Antriebsbewegungen, von der Griffzange ausgehend, über Rohrantriebe durch ein Trokar hierdurch an den Nähkopf 1 vorgegeben und in die Näh- sowie Umsetzbewegungen umgewandelt.

Vorteilhaft für den Chirurgen ist, wenn das eigentlich Nähen einhändig durchgeführt werden kann und mit der anderen Hand währenddessen Steuerbewegungen, falls nötig, erfolgen können. Somit muß der Nadelumsetzvorgang konfusionsfrei entkoppelt werden. Dies ist durch den fußbedienten, pneumatisch- mechanischen Antrieb an der Griffzange möglich.

Zu diesem Zweck ist am Oberteil der Griffzange 10 ein Miniatur-Pneumatikzylinder 13 angebaut, dessen Kolbenstange parallel zur Instrumentenachse 20 nach hinten über die in den Zylinder eingelassene Druckluft bewegt wird. Die Kolbenstange drückt dabei auf einen am Griffzangenoberteil drehbar gelagerten Hebel 15, der mit seinem einen Hebelteil über einen Bolzen 29 das Rohr 16 in die entgegengesetzte Richtung nach vorne schiebt. Die Klemmvorrichtung 8, 9 am starren Maulteil 2 der Figur 1 wird dadurch geschlossen, so daß Form- und Kraftschluß mit der eingespannten Nadel 4 besteht. Ein solcher Zustand kann aufrecht erhalten weren, wenn ständig Druckluft auf den Kolben im Zylinder 13 wirkt. Dieser Zustand ist unter Umständen hilfreich. Der Chirurg kann dann nämlich Gewebe auf die nur am starren Maulteil 2 eingespannte Nadel 4 aufschieben. Um diesen Zustand aufrecht erhalten zu können, führt die Druckluftzuleitung über einen einrastbaren Pedalschalter 12, den der Chirurg oder eine unmittelbar beim Chirurgen stehende Assistenzperson mit einem Fuß bedienen kann. Von diesem Pedalschalter 12 führt die Druckluftleitung weiter an den am Oberteil der Griffzange 10 angebrachten Druckluftanschlußstutzen 33 von dem aus eine Druckluftleitung zum Pneumatikzylinder 13 führt.

Wird der Pedalschalter 12 etwas weiter durchgetreten als die Einraststellung, so geht er zurück und die Druckluftzuführung wird gesperrt. Die Druckluft im Pneumatikzylinder 13 kann dann entweichen. Über eine über das Rohr 16 am nahen Ende bei der Griffzange 10 gestülpte und verankerte Rückhohlfeder wird dann das Rohr 16 selbständig zurückgezogen und die am starren Maulteil 3 eingespannte Nadelspitze 5 freigegeben.

Der Drehpunkt des Hebels 15 zwischen der Achse der Kolbenstange und der Instrumentenachse 20 bzw. die wirksamen Hebel zwischen Kolbenstange und Hebeldrehpunkt sowie Hebeldrehpunkt und Bolzen 29 sind einstellbar. Dadurch ist die Kraft auf das Rohr 16 eingeschränkt, und unnötige Krafteinwirkung auf den Kopf des Nähinstrumentes wird vermieden.

Für weitere Öffnungswinkel α max wird in Figur 4 nochmals die Modifikation von Figur 2 vorgestellt.Am drehbar gelagerten Ende des beweglichen Maulteils 3 befindet sich dort konzentrisch zur Drehachse 22 ein Zahnkranz 23, in den eine hin- und herbewegliche Zahnstange 24 eingreift und je nach Bewegungsrichtung und Länge der Bewegung das bewegliche Maulteil innerhalb des maximalen Öffnungswinkels α max mehr oder weniger weit auf und zuklappt. Da der Winkel α max konstruktiv sehr groß eingestellt werden kann, ist für ein solches Nähinstrument notwendig, daß dazu gekrümmte Nähnadel 4 verwendet werden. Hat die Nadel 4 eine Krümmung wie die Bahn 25 der Klemmvorrichtung 6,7, dann sticht sie lediglich durch das Gewebe oder die Gefäßwand und reißt nicht noch zusätzlich ein oder an.

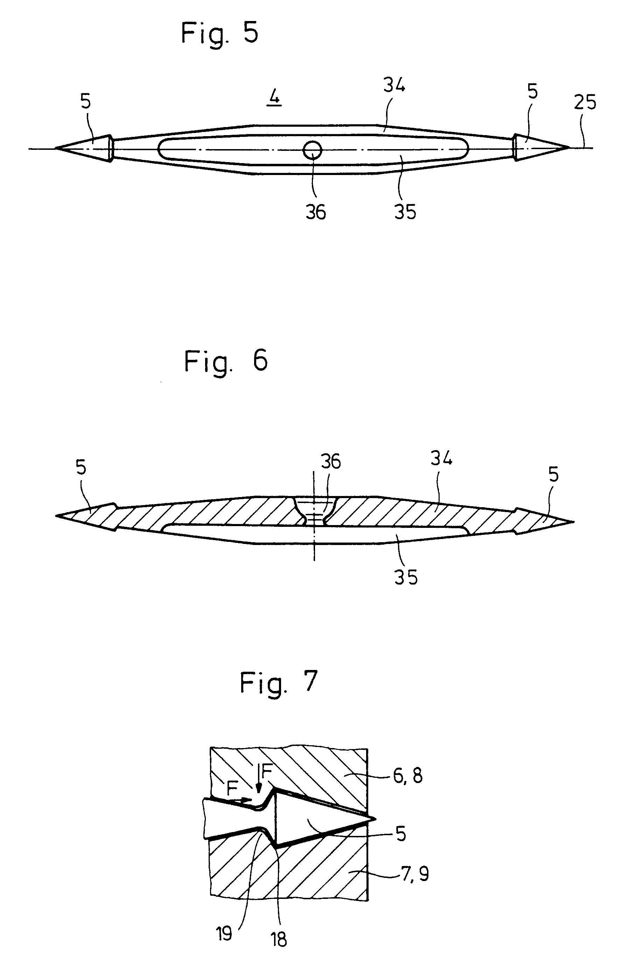

In Figur 5, 6 und 7 wird die in Figur 1 und 2 gestrichelt angedeutete Nadel 4 in Draufsicht, Längsschnitt und Spitzenausschnitt gezeigt.Die Nähnadel 4 ist schiffchenförmig und symmetrisch zu ihrer Längsachse 25. An beiden Enden hat sie gleichförmige Spitzen 5, die wulstartig an den Nadelkörper 36 anschließen. Im Nadelkörper 34 selber befindet sich eine Längshöhlung 35 mit der Querbohrung am Boden, dem Nadelöhr 26. Dort insbesondere sind die Kanten alle abgerundet. Der Nähfaden endet an dieser Stelle und ist dort angeknotet, ohne daß er übersteht. Ferner legt er sich beim Nähvorgang in die Längshöhlung 35, so daß dadurch Gewebeeinrisse beim Durchstechen vermieden werden.

Figur 7 hebt die Umfassung der Nadelspitze 5 durch die beiden Hälften 6, 8; 7, 9 der Klemmvorrichtungen hervor. Durch den kegelstumpfförmigen Übergang von Wulst 18 zum Nadelkörper setzen dort keine zu großen Kräfte an, so daß durch den Form- und Kraftschluß einerseits eine definierte Ausrichtung der Nadel 4 erreicht wird und andererseits ein Herausziehen oder ein Eindrücken der Nadel 4 mit vorgebbarem Kraftaufwand erfolgen kann. Die anschaulich wirkenden Kraftrichtungen um die Taille 19 der eingespannten Nadel 4 sind durch die senkrecht auf einanderstehenden Pfeile F angedeutet.

Im folgenden soll ein Nähvorgang beschrieben und der Nadelumsetzung dabei erläutert werden:
Zunächst wird die an einem Ende des Fadens angeknotete Nähnadel 4 längs zwischen die beiden zusammengeklappten oder beinahe zusammengeklappten Maulteile 2, 3 gelegt und eventuell an ihrer einen Spitze zwischen die beiden gerippten Flächen 30 geklemmt. Dann wird das Nähinstrument durch den Instrumentenkanal an den Nähort geführt. Dabei wird der Nähfaden mit durch die Rohre 16, 27 nachgezogen. Am Nähort wird das bewegliche Maulteil 3 aufgeklappt und die Nähnadel 4 mit Hilfe eines über einen anderen Instrumentenkanal eingeführten Halteinstruments in eine der Klemmvorrichtungen eingesetzt, vorzugsweise im starren Maulteil 2, da dieses von außen auf- und zuklappbar ist. Jetzt kann ein erster Stich durch zu nähendes Gewebe durchgeführt werden. Danach wird das bewegliche Maulteil 3 in Richtung des Maulteils 2 geklappt, bis schließlich die noch freie Nadelspitze 5 in der Klemmvorrichtung 6, 7 einrastet. Die Nadel 4 wird dann von der Klemmvorrichtung 8, 9 über Betätigen des Pedalschalters 12 freigegeben und zusammen mit dem Nähfaden vollends durch den Stich gezogen. Der Nähprozeß kann entsprechend durch Stich und Nadelumsetzen fortgeführt werden. Bei Bedarf ist aber auch nach jedem Stich und Fadendurchzug eine Knotung durchführbar, wobei das Straffziehen des Knotens eventuell mit Hilfe eines zusätzlichen Instruments erfolgt.

Die Dimensionen des Nähkopfes eines fertiggestellten Prototypen der beschriebenen Erfindung konnten wunschgemäß klein gehalten werden. Die Länge des Nähkopfes war 17 mm, die Länge des drehbaren Maulteils 12 mm. Dabei hatten die beiden Maulteile jeweils nur eine Dicke von etwa 1,5 mm. Der Nähkopf wurde durch einen Instrumentenkanal durchgeführt und über die Antriebseinrichtung aus koaxialen, ineinander laufenden Rohren 16, 27, 28 betätigt. Die lichte Weite des Instrumentenkanals oder Trokars war 5 mm.

Der erwartete Vorteil der einfachen Handgriffabfolge an der Griffzange und die Entkopplung des Näh- und Nadelumsetz-Vorgangs hat sich erfült.

### Bezugszeichenliste

- 1: Maulbereich, Nähkopf
- 2: feststehendes Maulteil
- 3: bewegliches Maulteil
- 4: Nadel
- 5: Nadelspitze
- 6: feststehende Hälfte
- 7: über Federkraft angepreßte Hälfte
- 8: feststehende Hälfte
- 9: bewegliche Hälfte
- 10: Griffzange
- 11: beweglicher Teil
- 12: Pedalschalter
- 13: Miniaturpneumatikzylinder
- 14: Achse des Miniaturpneumatikzylinders
- 15: Umlenkhebel
- 16: Rohr
- 17: Rückholfeder
- 18: Wulst
- 19: Taille
- 20: Nähinstrumentenachse, Rohrachse, Achse
- 21: Hebel
- 22: Drehachse
- 23: Zahnkranz
- 24: Zahnstange
- 25: Bahn, Achse
- 26: Feder
- 27: Rohr
- 28: Instrumentenrohr
- 29: Bolzen
- 30: gerippte Fläche
- 31: Schiebevorrichtung
- 32: Blech
- 33: Anschlußstutzen
- 34: Nadelkörper
- 35: Längshöhlung
- 36: Nadelöhr

## Patentansprüche

1. Chirurgisches Nähinstrument für die minimal invasive Chirurgie, das auf dem fernen Ende einer durch ein Trokar führenden Antriebseinrichtung (16, 27, 28) aus koaxial ineinander laufenden Rohren (16, 27, 28) aufgesetzt ist und über eine am nahen Ende der Antriebseinrichtung (16, 27, 28) aufgesetzte Griffzange (10) mit einem beweglichen Griffteil (11) über Hebelwirkung betätigt wird, mit den weiteren Merkmalen:
- das Nähinstrument hat ein bewegliches (3) und ein feststehendes Maulteil (2) mit je einer Klemmvorrichtung (6, 7; 8, 9), wobei die Klemmvorrichtung (6, 7) am beweglichen Maulteil (3) über einen Federmechanismus selbsttätig geschlossen gehalten wird und die Klemmvorrichtung (8, 9) am feststehenden Maulteil (2) über die Griffzange (10) geschlossen oder gelöst werden kann;
- eine in den Klemmvorrichtungen (6, 7; 8, 9) einzuspannende Nadel (4) weist an ihren beiden Enden je eine Spitze (5) mit Wulst (18) und Taille (19) auf, um die sich die Klemmvorrichtungen (6, 7; 8, 9)form- und kraftschlüssig legen und die Nadel (4) ein- oder beidseitig hält;
- die Nadel (4) hat zwischen beiden Spitzen (5)ein Nadelöhr (36) zur Verknotung eines Nähfadenendes;
dadurch gekennzeichnet, daß
- die Längsachse (25) der Nadel (4) durch ihre beiden Spitzen die Form der Bahn (25) hat, die die Klemmvorrichtung (6, 7) des beweglichen Maulteils (3) beim Bewegen beschreibt;
- die Klemmvorrichtungen (6, 7;8, 9) je aus einem feststehenden Teil (6, 8) und einem beweglichem Teil (8, 9) bestehen und die Teile (6, 7;8, 9) jeweils die ergänzende Hälfte des Abdrucks der Nadelspitze (5) aufweisen;
- die Bewegung des beweglichen Maulteils (3) einhändig über den beweglichen Teil (11) an der Griffzange (10) und das übertragende Rohr (27) der Antriebseinrichtung (16, 27, 28) erfolgt;
- die Bewegung der beweglichen Hälfte (9) der Klemmvorrichtung (8,9) über einen Pedalschalter (12) erfolgt, der mit seinem Ausgang an eine an der Griffzange (10) angebrachten, kraftabgestimmten Antriebseinrichtung (13,14,15) angeschlossen ist;
- an der Griffzange (10) an ihrem starren Teil ein Miniaturpneumatik-Zylinder (13) mit seiner Achse (14) parallel zur einlaufenden Achse (20) des Rohres (16) angebracht ist und zwischen beiden Achsen (14,20) ein Umlenkhebel (15) derart drehbar gelagert ist, daß zum Angriffspunkt der Schubstange des Miniaturpneumatik-Zylinders (13) am Umlenkhebel (15) und zum Angriffspunkt des nahen Endes des Rohres (16) am Umlenkhebel (15) jeweils ein vorgegeben wirksamer Hebelarm besteht;
- die in den Miniaturpneumatik-Zylinder (13) eingelassene Luft vorbestimmten Druckes das Rohr (16) nach vorne drückt und damit die Nadelhalterung (8,9) schließt oder geschlossen hält, und im Falle der aus dem Zylinder (13) abgelassenen Luft die Kolbenstange am Zylinder (13) über eine dort eingebaute Feder und das Rohr (16) über eine über sie gestülpte Rückhol-Feder (17) zurückzieht und damit die Nadelhalterung (8,9) öffnet oder geöffnet hält.

2. Chirurgisches Nähinstrument nach Anspruch 1,
dadurch gekennzeichnet, daß
Wulst (18) und Taille (19) an der Negativform bei der Einspannvorrichtung (6,7;8,9) abgerundet sind und über eine kurze Kegelstumpfmantelfläche glatt ineinander übergehen, wodurch das Herausziehen und Eindrücken der Nadel (4) beschädigungslos und kraftbestimmt durchführbar ist.

3. Chirurgisches Nähinstrument nach Anspruch 1,
dadurch gekennzeichnet, daß
das bewegliche Griffzangenteil (11) zum Bewegen des beweglichen Maulteils (3) arretierbar ist, wodurch eine feste Stellung der Maulteile (2, 3) zueinander eingestellt und wieder freigegeben werden kann.

4. Chirurgisches Nähinstrument nach einem der Ansprüche 1 bis 3,
dadurch gekennzeichnet, daß
- beide Maulteile (2,3) senkrecht von der Instrumentenachse (20) wegweisen;
- die Achse (25) der eingespannten geraden Nadel (4) parallel zur Instrumentenachse (20) liegt;
- die bewegliche Hälfte (9) der Klemmvorrichtung (8,9) am feststehenden Maulteil (2) über einen daran drehbar gelagerten Hebel (21) an die feststehende Hälfte (8) angepreßt oder von ihr weggedreht werden kann.

5. Chirurgisches Nähinstrument nach Anspruch 1 bis 3,
dadurch gekennzeichnet, daß
- das feststehende Maulteil (2) in Verlängerung zur Instrumentenachse (20) liegt und das bewegliche Maulteil (3) um eine Drehachse (22) senkrecht dazu gelagert ist, so daß dieses über einen Hebel in einem vorgegebenen Winkelbereich auf das feststehende Maulteil (2) zu- oder aufgeklappt werden kann.

6. Chirurgisches Nähinstrument nach Anspruch 5,
dadurch gekennzeichnet, daß
das bewegliche Maulteil (3) an seiner Drehachse (22) mit einem dazu konzentrischen Zahnkranz (23) versehen ist, in den eine axial verschiebbare Zahnstange (24) eingreift.

7. Chirurgisches Nähinstrument nach den Ansprüchen 4, 5 und 6,
dadurch gekennzeichnet, daß
anstelle der beiden Klemmvorrichtungen (6,7;8,9) an einem Maulteil (2 bzw. 3) eine Klammerhaltevorrichtung und an dem anderen Maulteil (3 bzw. 2) eine Gesenkvorrichtung eingebaut ist, so daß Gewebe oder Gefäßteile chirurgisch geklammert werden können.

8. Chirurgisches Nähinstrument nach den Ansprüchen 4, 5 und 6
dadurch gekennzeichnet, daß
die freien Enden der Maulteile (2, 3) gerippt sind und dadurch das Nähinstrument bei nicht eingespannter Nadel (4) ein Greifinstrument ist.

## Claims

1. Surgical stitching instrument for minimally invasive surgery, which instrument is mounted on the far end of a drive means (16, 27, 28), which passes through a trocar and is formed from tubes (16, 27, 28) extending coaxially into one another, and said instrument is actuated by lever action via a pair of gripping tongs (10), which is mounted on the near end of the drive means (16, 27, 28) and has a displaceable gripping part (11), having the additional features:
- the stitching instrument has a displaceable mouthpiece (3) and a stationary mouthpiece (2), each having a clamping device (6, 7; 8, 9), the clamping device (6, 7) on the displaceable mouthpiece being automatically kept closed via a spring mechanism, and the clamping device (8, 9) on the stationary mouthpiece (2) being closable or releasable via the gripping tongs;
- a needle (4), which is to be clamped in position in the clamping devices (6, 7; 8, 9), has a point (5) at each of its two ends with a bead (18) and reduced portion (19), around which point the clamping devices (6, 7; 8, 9) are placed in a form- and force-locking manner, and the needle (4) is retained at one or both ends;
- the needle (4) has a needle eye (36) between both points (5) for the knotting of one end of the stitching thread;
characterised in that
- the longitudinal axis (25) of the needle (4) through its two points has the configuration of the path (25) which the clamping device (6, 7) of the displaceable mouthpiece (3) describes during movement;
- the clamping devices (6, 7; 8, 9) each comprise a stationary portion (6, 8) and a displaceable portion (8, 9), and the portions (6, 7; 8, 9) each have the complementary half of the shape of the needle point (5);
- the movement of the displaceable mouthpiece (3) is effected with one hand via the displaceable part (11) of the gripping tongs (10) and via the transmitting tube (27) of the drive means (16, 27, 28);
- the movement of the displaceable half (9) of the clamping device (8, 9) is effected via a foot-pedal switch (12), which communicates by its output with a power-adapted drive means (13, 14, 15) mounted on the gripping tongs (10);
- a miniature pneumatic cylinder (13) is mounted on the rigid part of the gripping tongs (10) with its axis (14) parallel to the incoming axis (20) of the tube (16), and a shift lever (15) is rotatably mounted between both axes (14, 20) in such a manner that there is a prescribed active lever arm at both the point of engagement between the push-rod of the miniature pneumatic cylinder (13) and the shift lever (15) and the point of engagement between the near end of the tube (16) and the shift lever (15);
- the air, which is admitted into the miniature pneumatic cylinder (13) and has a predetermined pressure, urges the tube (16) forwardly and thereby closes or keeps closed the needle holder (8, 9) and, when the air is released from the cylinder (13), such air withdraws the piston rod at the cylinder (13) via a spring incorporated there and withdraws the tube (16) via a return spring (17), which is inverted over the former spring and thereby opens or keeps open the needle holder (8, 9).

2. Surgical stitching instrument according to claim 1, characterised in that bead (18) and reduced portion (19) are rounded on the negative form for the clamping device (6, 7; 8, 9) and pass smoothly into each other via a short truncated conical surface, whereby the withdrawal and insertion of the needle (4) can be effected without any damage and in a power-determined manner.

3. Surgical stitching instrument according to claim 1, characterised in that the displaceable part (11) of the gripping tongs is lockable for the movement of the displaceable mouthpiece (3), whereby a fixed position for the mouthpieces (2, 3) relative to each other can be set and released again.

4. Surgical stitching instrument according to one of claims 1 to 3, characterised in that
- both mouthpieces (2, 3) are orientated away from the instrument axis (20) perpendicularly;
- the axis (25) of the clamped rectilinear needle (4) lies parallel to the instrument axis (20);
- the displaceable half (9) of the clamping device (8, 9) on the stationary mouthpiece (2) can be pressed against the stationary half (8) or rotated away from said stationary half via a lever (21), which is rotatably mounted thereon.

5. Surgical stitching instrument according to claims 1 to 3, characterised in that
- the stationary mouthpiece (2) lies in an extension of the instrument axis (20), and the displaceable mouthpiece (3) is mounted about a rotary axis (22) perpendicular thereto, so that said displaceable mouthpiece can be pivoted towards or onto the stationary mouthpiece (2) at a prescribed angular range via a lever.

6. Surgical stitching instrument according to claim 5, characterised in that the displaceable mouthpiece (3) is provided at its rotary axis (22) with a toothed ring (23), which is concentric therewith and in which an axially displaceable toothed rod (24) engages.

7. Surgical stitching instrument according to claims 4, 5 and 6, characterised in that, instead of the two clamping devices (6, 7; 8, 9), a bracket holding device is installed on a mouthpiece (2 or 3) and a die-stamping device is installed on the other mouthpiece (3 or 2), so that tissue or parts of vessels can be clamped surgically.

8. Surgical stitching instrument according to claims 4, 5 and 6, characterised in that the free ends of the mouthpieces (2, 3) are ribbed, and the stitching instrument is thereby a gripping instrument when the needle (4) is not clamped in position.

## Revendications

1. Instrument de suture chirurgicale pour la chirurgie invasive minimale, qui est placée à l'extrémité éloignée d'un dispositif de commande (16, 27, 28) agissant par un trocart et composé de tubes (16, 27, 28) se déplaçant coaxialement l'un dans l'autre et qui est actionné par l'effet d'un levier sur une pince de prise (10) placée à l'extrémité voisine du dispositif de commande (16, 27, 28) ayant une partie de pince mobile (11), instrument comportant l'instrument de suture a une partie de mâchoire (3) mobile et une partie de mâchoire (2) fixe avec chacune un dispositif de verrouillage (6, 7 ; 8, 9), le dispositif de verrouillage (6, 7) sur la partie de mâchoire mobile (3) étant maintenu automatiquement fermé par un mécanisme de ressort et le dispositif (8, 9) sur la partie de mâchoire fixe (2) pouvant être fermé ou déclenché par la pince de prise (10), une aiguille (4) à insérer dans les dispositifs de blocage (6, 7 ; 8, 9) comporte à chacune de ses deux extrémités une pointe (5) avec un bourrelet (18) et un col (19), autour desquels se mettent en place les dispositifs de blocage (6, 7 ; 8, 9) par une liaison par la forme et la force et l'aiguille est maintenue sur un ou des deux côtés, caractérisé en ce que l'axe longitudinal (25) de l'aiguille (4) a à travers ses deux pointes la forme de la trajectoire (25), qui décrit le dispositif de blocage (6, 7) de la partie de mâchoire mobile (3) lors de son mouvement, les dispositifs de blocage (6, 7 ; 8, 9) se composent chacun d'une partie fixe (6, 8) et d'une partie mobile (8, 9) et les parties (6, 7 ; 8 , 9) présentent respectivement la moitié complète de la pression d'empreinte de la pointe d'aiguille (5), le mouvement de la partie de mâchoire mobile (3) a lieu avec une main sur la partie mobile (11) de la pince de prise (10) et sur le tube (27) de transmission du dispositif de commande (16, 27, 28), un mouvement de la moitié mobile (9) du dispositif de blocage (8, 9) a lieu par un commutateur à pédale (12), qui est raccordé par sa sortie à un dispositif de commande (13, 14, 15) appliqué à la pince de prise (10) et synchronisé par la force, sur la pince de prise (10) sur sa partie fixe est appliqué un vérin pneumatique miniature (13) avec son axe (14) parallèle à l'axe (20) d'introduction du tube (16) et un levier de renvoi (15) est monté mobile en rotation entre les deux axes (14, 20) de manière à constituer un bras de levier donné efficient pour le point d'attaque de la tige de poussée du vérin pneumatique miniature (13) sur le levier de renvoi (15) et pour le point d'attaque de l'extrémité proche du tube (16) sur le levier de renvoi (15), l'air de pression pré-établi introduit dans le vérin pneumatique miniature (13) presse le tube (16) vers l'avant et donc ferme ou maintient fermé le support d'aiguille (8, 9) et dans le cas où l'air évacué du vérin (13) la tige de piston du vérin (13) est tirée en arrière par un ressort installé à cet endroit et le tube (16) par un ressort de renvoi (17) placé sur lui et donc le support d'aiguille (8, 9) ouvre ou se maintient ouvert.

2. Instrument chirurgical de suture selon la revendication 1, caractérisé en ce que le bourrelet (18) et le col (19) sont arrondis sur une forme négative dans le cas du dispositif de blocage (6, 7 ; 8, 9) et se convertissent l'un dans l'autre de manière lisse par une surface enveloppe de tronc de cône, raison pour laquelle le retrait et l'insertion de l'aiguille (4) sont réalisables sans dommage et déterminées par la force.

3. Instrument chirurgical de suture selon la revendication 1 , caractérisé en ce que la partie de pince de prise mobile (11) peut être arrêtée pour le mouvement de la partie de mâchoire mobile (3), par suite de quoi une position fixe des parties de mâchoire (2, 3) peut être ajustée l'une par rapport à l'autre et peut être à nouveau libérée.

4. Instrument chirurgical de suture selon une des revendications 1 à 3, caractérisé en ce que les deux parties de mâchoire (2, 3) ont une direction perpendiculaire à l'axe de l'instrument (20) l'axe (25) de l'aiguille droite introduite (4) est parallèle à l'axe de l'instrument (20), la moitié mobile (9) du dispositif de blocage (8, 9) sur la partie fixe de mâchoire (2) est pressée contre la moitié fixe (8) par un levier (21) monté mobile en rotation ou peut être stoppé par lui.

5. Instrument chirurgical de suture selon les revendications 1 à 3, caractérisé en ce que la partie fixe de mâchoire (2) se situe en prolongement par rapport à l'axe de l'instrument (20) et la partie mobile de mâchoire (3) est montée autour d'un axe de rotation (22) perpendiculairement à celui-ci, de sorte que cette partie (3) peut être ouverte ou fermée en basculant un levier dans un domaine angulaire préfixé sur la partie fixe de mâchoire (2).

6. Instrument de suture chirurgical selon la revendication 5, caractérisé en ce que la partie de mâchoire mobile (3) est munie sur son axe de rotation (22) d'une couronne dentée (23) concentrique à cette dernière, dans laquelle s'engrène une crémaillère (24) déplaçable axialement.

7. Instrument de suture chirurgical selon les revendications 4, 5 et 6, caractérisé en ce qu'au lieu des deux dispositifs de blocage (6, 7 ; 8, 9) sur une des parties de mâchoire (2 ou 3) est monté un dispositif de support d'agrafe et sur l'autre partie de mâchoire (3 ou 2) un dispositif de matriçage, de façon que les tissus ou les vaisseaux sanguins puissent être agrafés chirurgicalement.

8. Instrument de suture chirurgical selon les revendications 4, 5 et 6, caractérisé en ce que les extrémités libres des parties de mâchoire (2, 3) sont cannelées et, par suite l'instrument de suture dans le cas de manque d'aiguilles (4) insérées est un instrument de prise.
